# DEMANDE DE BREVET EUROPEEN

(11) **EP 0 713 919 A1**
(43) Date de publication de la demande: **29.05.1996**
(21) Numéro de dépôt: 95402596.1
(22) Date de dépôt: 17.11.1995
(51) Int. Cl.: C12Q 1/04

(54) **Moyens pour le traitement de prélèvements susceptibles de renfermer des microorganismes pathogènes**

(30) Priorité: 17.11.1994 FR 9413780
(71) Demandeur: FDM PHARMA, F-06560 Sophia Antipolis (FR); INSTITUT ALFRED FOURNIER, F-75014 Paris (FR)
(72) Inventeur: Alonso, Jean-Michel, F-78180 Montigny le Bretonneux (FR); Sednaoui, Patrice, F-92330 Sceaux (FR); Janin, Nicole, F-75015 Paris (FR); Van Rompaey, Dominique, F-75004 Paris (FR)
(74) Mandataire: Peaucelle, Chantal

(57) **Abrégé**

L'invention vise un procédé pour le traitement de prélèvements à analyser susceptibles de renfermer des microorganismes pathogènes, dans lequel on utilise un milieu élaboré à partir d'une solution tamponnée et d'au moins un agent ayant un effet cryoprotecteur intracellulaire vis-à-vis des microorganismes, en combinaison avec du lait et/ou un dérivé de lait.

## Description

L'invention a pour objet des moyens, procédés et milieux, pour le traitement de prélèvements à analyser, contaminés par des microorganismes pathogènes.

Elle vise plus spécialement des moyens permettant de conserver qualitativement et quantitativement les microorganismes dans l'état où ils se trouvent au moment du prélèvement.

On sait que la conservation de microorganismes fait appel, de manière générale, à des techniques de congélation et/ou de lyophilisation.

Afin de remédier aux effets du choc osmotique subi par le microorganisme lors de l'application de ces techniques, il est habituel d'ajouter à la culture de microorganismes, ou aux cellules en suspension, un agent cryoprotecteur. Il s'agit le plus fréquemment de glycérol. Ce composé pénètre dans la cellule du microorganisme et se substitue à l'eau intracellulaire, ce qui permet, lors des étapes de congélation/décongélation, de réduire les différences de pression osmotique exercées sur les membranes, et par conséquent, leur destruction.

Des cryoprotecteurs extracellulaires, c'est-à-dire qui ne pénètrent pas dans la cellule, ont également été décrits. Il s'agit par exemple de la polyvinylpyrrolidone, de sucres simples comme le lactose, le glucose ou le saccharose, du lait ou de dérivés du lait, ou encore d'hétérosides comme le mannitol ou le sorbitol. Ces produits ont pour effet de protéger les microorganismes de processus de protéolyses.

La mise en oeuvre de ces moyens permet d'obtenir des résultats relativement satisfaisants pour la conservation de souches de microorganismes pures.

En revanche, ces techniques ne s'avèrent pas suffisamment performantes lorsqu'il s'agit de conserver des prélèvements biologiques ou du domaine agro-alimentaire, contaminés par des microorganismes pathogènes qui renferment donc ces microorganismes en mélange avec de nombreux autres types cellulaires. Ces produits sont en effet très labiles, ce qui nécessite la plupart du temps la réalisation d'analyses sur place.

Or, à l'heure actuelle, les analyses biologiques ont de plus en plus tendance à être centralisées. Ceci implique d'être en mesure de transporter les échantillons à analyser dans les centres d'analyse en les conservant dans l'état où ils se trouvent lors du prélèvement.

L'invention a pour but de fournir une solution à ces problèmes en proposant de nouveaux moyens basés sur l'action synergique mise en évidence en utilisant certains types d'agents cryoconservateurs en combinaison dans un milieu donné.

L'invention vise donc un procédé de traitement de prélèvements susceptibles de renfermer des microorganismes pathogènes, permettant de les conserver et de les préserver de toute altération par les composés mêmes de l'échantillon et d'atteindre ainsi une viabilité de l'ordre de 100 %.

Selon un autre aspect, l'invention a pour but l'application de ce procédé à l'analyse de ces prélèvements à des fins diagnostiques ou à titre de contrôle et de prévention dans le domaine agro-alimentaire.

Le procédé de conservation de micro-organismes pathogènes selon l'invention est caractérisé en ce qu'il comporte l'utilisation d'un milieu élaboré à partir d'une solution aqueuse tamponnée et d'au moins un agent ayant un effet cryoprotecteur intracellulaire vis-à-vis des microorganismes, en combinaison avec du lait et/ou un dérivé de lait.

L'utilisation d'un tel milieu permet de conférer une viabilité élevée au prélèvement, assurant ainsi une survie des microorganismes atteignant généralement 100 %, et ce même dans le cas de microorganismes pathogènes fragiles qui nécessitent jusqu'à présent un transport rapide au Laboratoire qui les mettra en culture, ou avec des échantillons à faibles concentrations en microorganismes pathogènes. Ce milieu permet également d'empêcher la multiplication des microorganismes présents dans les prélèvements. Il est alors possible de quantifier avec une grande fiabilité les différentes populations pathogènes du prélèvement.

De manière préférée, le ou les agents cryoprotecteurs intracellulaires sont utilisés à raison de 15 à 35 % (v/v), notamment de 20 à 30 % environ, et le lait ou les dérivés de lait à raison de 3 à 7 % (v/v) et notamment de 5 % environ.

Un agent cryoprotecteur particulièrement préféré est constitué par le glycérol. Des résultats satisfaisants peuvent être également obtenus avec les cryoprotecteurs tels que le diméthyl sulfoxyde ou le polyéthylèneglycol.

Le lait est plus spécialement utilisé sous forme de poudre et en particulier de granulés en poudre. Il s'agit avantageusement de lait écrémé.

L'utilisation en combinaison de ces deux types de produits en milieu tamponné conduit à un effet synergique sur la viabilité des microorganismes pathogènes, et ce bien qu'ils se trouvent dans leur milieu de prélèvement, c'est-à-dire avec des produits pouvant exercer une action lytique à leur encontre.

Le milieu de conservation renfermant les produits à effet synergique peut être enrichi en sels minéraux, vitamines et autres éléments ou produits convenant pour le type de microorganisme présent dans le prélèvement, ainsi qu'en inhibiteurs d'enzymes.

La solution tamponnée renfermant les différents composés mentionnés ci-dessus est avantageusement constituée par une solution aqueuse de NaCl 0,15 M.

Selon un mode de mise en oeuvre avantageux, le procédé de l'invention comprend les étapes de mélange de l'échantillon avec le milieu défini ci-dessus et de conservation pendant une durée et à une température appropriées au type de microorganisme susceptible de se trouver dans l'échantillon à analyser.

Ainsi la température doit être inférieure à -30°C pour bloquer la prolifération des microorganismes d'une part, et pour préserer la viabilité de ces derniers d'autre part.

Le prélèvement ainsi traité peut être conservé plusieurs jours ce qui permet, après conditionnement, son transport jusqu'au laboratoire où l'analyse qualitative et quantitative doit être effectuée.

L'invention vise donc également un récipient étanche comportant un échantillon additionné d'un milieu tel que défini ci-dessus, le récipient assurant les conditions de température requises pour la conservation de l'échantillon dans l'état où il se trouvait au moment de son prélèvement.

Le procédé de l'invention est particulièrement approprié pour la survie de microorganismes pathogènes tels que bactéries, champignons, virus pathogènes ou contaminants de produits agro-alimentaires.

Ce procédé constitue en particulier une solution au problème de la viabilité des bactéries telles que les bactéries aérobies pathogènes, à Gram positif ou à Gram négatif, considérées comme très fragiles, des Streptocoques, Staphylocoques, des Hemophilus, des Listeria, des Neisseria, des Proteus, des Pseudomonas, des Chlamydia, des Escherichia Coli urinaires ou des Salmonelles ou encore des bactéries anaérobies.

On notera notamment l'intérêt de l'utilisation du milieu de conservation selon l'invention pour le transport de prélèvements contenant des bactéries de la flore respiratoire. Les prélèvements d'expectorations étant notoirement considérés comme très fragiles et non transportables dans des délais excédant quelques heures.

Le procédé de l'invention est également particulièrement avantageux pour effectuer avec précision des analyses de Candida et d'autres levures potentiellement pathogènes.

L'invention fournit ainsi des moyens pour analyser avec une grande fiabilité des prélèvements d'origine humaine, animale ou végétale ou pour effectuer des contrôles dans le domaine agro-alimentaire, par exemple dans les produits carnés, les conserves et les produits laitiers.

On rapporte dans les exemples qui suivent d'autres caractéristiques et avantages de l'invention à titre purement illustratif.

### Exemple 1 : Etude de la viabilité de microorganismes pathogènes dans des échantillons contaminés.

On utilise des souches de Streptococcus pneumoniae et de Hemophilus influenzae isolées depuis moins de 48 h d'expectorations.

La souche de S.pneumoniae est cultivée sur gélose au sang et celle de H.influenzae sur gélose chocolat.

Les géloses sont incubées à 37°C pendant 18 h sous atmosphère de CO₂.

On prépare un inoculum en utilisant un liquide d'expectoration stérile (pool provenant de crachats adressés pour la recherche de mycobactéries: 10 tubes de 2 ml autoclavés à 120°C, 20 min).

Chaque tube est ensemencé avec un inoculum quantifié de l'une ou l'autre des deux souches bactériennes (nombre de CFU/ml).

A partir de chaque échantillon artificiellement contaminé, des numérations sont pratiquées au temps zéro (T_{O}) et après 24 h.

On rapporte ci-après les résultats de deux essais.

### Essai 1

On utilise, comme inocula, des souches de S.pneumoniae à raison de 2,1 (± 0,57)x10⁷ CFU/ml et de H.influenzae à raison de 6,85 (± 0,53)x10⁷ CFU/ml.

Pour chaque souche, 5 tubes d'expectorations (2ml/tube) sont ensemencés avec 10 microlitres de la suspension bactérienne et mélangés au Vortex.

Ensuite, on conserve 0,5 ml de chaque échantillon
(1) à +4°C, ou
(2) à -20°C pendant 2 h, puis en carboglace en conteneur, par exemple en polystyrène standard, ou
(3) on ajoute du glycérol jusqu'à une concentration finale de 30 % (v/v), puis on traite l'échantillon comme sous (2), ou
(4) on le laisse à température ambiante.

Les résultats obtenus après 24 h selon le milieu et les conditions de conservation (nombre de CFU/10 µl) sont donnés dans le tableau 1.

Les colonnes (1), (2), (3), (4), correspondent respectivement aux conditions rapportées ci-dessus.

**TABLEAU 1**

| S.pneumoniae | | | | | |
|---|---|---|---|---|---|
| N°du tube | Tₒ | (1) | (2) | (3) | (4) |
| 1 | env.10000 | 1 | 0 | env.300 | 74 |
| 2 | env.10000 | 2 | 0 | env.300 | 27 |
| 3 | env.10000 | 1 | 0 | 110 | 70 |
| 4 | env.10000 | 1 | 1 | 80 | 30 |
| 5 | env.10000 | 2 | 0 | 130 | 53 |

| H.influenzae | | | | | |
|---|---|---|---|---|---|
| N° du tube | Tₒ | (1) | (2) | (3) | (4) |
| 6 | 10000 env. | 0 | 0 | env.800 | 0 |
| 7 | 10000 env. | 0 | 0 | env.500 | 0 |
| 8 | 10000 env. | 0 | 0 | env.500 | 0 |
| 9 | 10000 env. | 0 | 0 | env.300 | 0 |
| 10 | 10000 env. | 0 | 0 | env.300 | 0 |

On constate que l'addition de glycérol et la conservation à -20°C, permet d'obtenir une faible survie des deux types de bactéries, d'environ 1 à 3 % pour S. pneumoniae et de l'ordre de 3 à 8 % pour H.influenzae.

La survie des bactéries est en revanche nulle, ou pratiquement nulle, dans des conditions de conservation à +4°C ou à -20°C pendant 24h sans utilisation de cryoprotecteur. A température ambiante, on observe une très faible survie seulement dans le cas de S.pneumoniae.

### Essai 2

On utilise un inoculum de 1x10⁶ CFU/ml, à raison de 10 µlitres/tube.

On réalise les essais suivants en double :
1) essais avec une concentration finale de 30 % en glycérol : on ajoute 0,3 ml de glycérol à 0,7 ml de crachat ;
2) essais avec une concentration finale de 30 % en glycérol et de 5 % en lait écrémé du commerce : on ajoute 0,3 ml de glycérol et 0,1 ml de lait écrémé sous forme de granulés en poudre à 50 % à 0,6 ml de crachat ;
3) essais avec une concentration finale de 5 % en lait écrémé du commerce : on ajoute 0,1ml de lait écrémé en poudre à 0,9 ml de crachat.

On laisse les échantillons 2 h à -20°C, puis 22 h dans de la carboglace, en boîte isotherme.

Les résultats relatifs à la survie des bactéries selon la composition du milieu (2 tubes/mesure) sont rapportés dans le tableau 2 (T₀ est d'environ 10000 CFU/litre).

**TABLEAU 2**

| S.pneumoniae | | |
|---|---|---|
| glycérol | lait écr. en poudre | lait écr. en poudre + glycérol |
| (0,8x1,3)x10⁴ | (0,75-1,25)x10⁴ | (1,8-8)x10⁴ |

| H.inluenzae | | |
|---|---|---|
| glycérol | lait écr. en poudre | lait écr. en poudre + glycérol |
| (0,7-5)x10⁴ | (0,9-1,4)x10⁴ | (6-6,3)x10⁴ |

L'examen de ces résultats met en évidence l'effet synergique exercé par l'utilisation en combinaison d'un agent cryoprotecteur avec du lait.

### Exemple 2 : Application du procédé de l'invention au transport d'abcès secondaires.

On ajoute au prélèvement effectué, sous agitation un milieu de conservation constitué par une solution aqueuse tamponnée, renfermant 0,15 M de NaCl, du glycérol à raison de 30 %, et du lait écrémé en poudre sous forme de granulés, à raison de 5 %.

L'échantillon est placé dans un récipient assurant une température d'environ -30° C et transporté dans un laboratoire aux fins d'identification et de numération des bactéries.

### Exemple 3 : Application du procédé de l'invention à la conservation et au transport de prélèvements d'aliments.

A des fins de contrôle, on effectue à l'usine ou en chambre froide des prélèvements sur des produits carnés (tels que pâtés, rillettes, viande hachée) ou de produits lactés non fermentés (lait, crèmes, fromages frais). On ajoute aux prélèvements une solution aqueuse tamponnée, renfermant 0,15 M de NaCl, 30 % de glycérol ou 10 % de DM50 ou de mannitol et 5 % de lait écrémé. Les prélèvements sont congelés à - 80°C et placés dans des récipients renfermant de la carboglace et transportés jusqu'aux laboratoires de contrôle aux fins d'analyse.

### Exemple 4 : Application du procédé de l'invention à la recherche d'un agent phytopathogène.

On prélève une tige ou une racine de crucifère (comme le chou ou la salade), ou une pousse de peuplier ou d'orme et la traite comme décrit dans l'exemple 2, aux fins de transport et d'analyse.

## Revendications

1. Procédé pour le traitement de prélèvements susceptibles de renfermer des microorganismes à analyser pathogènes, caractérisé en ce qu'il comporte l'utilisation d'un milieu élaboré à partir d'une solution aqueuse tamponnée et d'au moins un agent ayant un effet cryoprotecteur intracellulaire vis-à-vis des microorganismes, en combinaison avec du lait et/ou un dérivé de lait.

2. Procédé selon la revendication 1, caractérisé en ce que le ou les agents cryoprotecteurs, tels que le glycérol ou le diméthyl sulfoxyde sont utilisés à raison de 15 à 35 % (v/v), notamment de 20 à 30 % environ et le lait ou les dérivés de lait, à raison de 3 à 7 % (v/v), et notamment de 5 % environ.

3. Procédé selon la revendication 2, caractérisé en ce que les agents cryoprotecteurs sont choisis parmi le glycérol, le diméthylsulfoxyde, l'éthylèneglycol, l'acétate d'ammonium ou de triéthylammonium.

4. Procédé selon l'une quelconque des revendications 1 à 3, caractérisé en ce que le lait est utilisé sous forme de poudre, en particulier de granulés en poudre.

5. Procédé selon la revendication 4, caractérisé en ce qu'il s'agit de lait écrémé.

6. Procédé selon l'une quelconque des revendications 1 à 5, caractérisé en ce que le milieu tamponné est formé par de l'eau renfermant 0,15 M de NaCl.

7. Procédé selon l'une quelconque des revendications précédentes, caractérisé en ce qu'il comprend les étapes de mélange de l'échantillon avec le milieu défini dans l'une des revendications 1 à 6, et de conservation pendant une durée et une température appropriées au type de microorganisme susceptible de se trouver dans l'échantillon à analyser.

8. Application du procédé selon l'une quelconque des revendications 1 à 7, à la conservation et au transport aux fins d'analyse de prélèvements biologiques d'origine humaine, animale ou végétale.

9. Application du procédé selon l'une quelconque des revendications 1 à 7, à la conservation et au transport aux fins d'analyse de prélèvements d'origine alimentaire.
